# EUROPEAN PATENT SPECIFICATION

(11) **EP 2 236 137 B1**
(45) Date of publication and mention of the grant of the patent: **24.06.2015**
(21) Application number: 09705558.6
(22) Date of filing: 29.01.2009
(51) Int. Cl.: A61K 31/351, A61K 31/381, A61P 1/16, C07D 309/10, C07D 409/10, C07H 7/04, C07H 15/203, C07H 15/26

(54) **PHARMACEUTICAL COMPOSITION FOR TREATMENT OF FATTY LIVER DISEASES**
PHARMAZEUTISCHE ZUSAMMENSETZUNG ZUR BEHANDLUNG VON FETTLEBERERKRANKUNGEN
COMPOSITION PHARMACEUTIQUE POUR LE TRAITEMENT DE STÉATOSES HÉPATIQUES

(30) Priority: 31.01.2008 JP 2008020731; 31.03.2008 JP 2008089638
(43) Date of publication of application: 06.10.2010
(73) Proprietor: Astellas Pharma Inc., Chuo-ku Tokyo 103-8411 (JP); Kotobuki Pharmaceutical Co., Ltd., Hanishina-gun, Nagano 389-0697 (JP)
(72) Inventor: KUROSAKI, Eiji, Tokyo 103-8411 (JP); TAKASU, Toshiyuki, Tokyo 103-8411 (JP); MAEDA, Noriaki, Tokyo 103-8411 (JP); YAMAZAKI, Shunji, Tokyo 103-8411 (JP)
(74) Representative: Hoffmann Eitle
(86) International application number: PCT/JP2009/051434
(87) International publication number: WO 2009/096455

(56) References cited:
- EP-A1- 1 782 828
- WO-A1-02/09757
- WO-A1-03/099836
- WO-A1-2004/013118
- WO-A1-2004/080990
- WO-A1-2006/009149
- WO-A1-2007/007628
- WO-A1-2007/114475
- WO-A1-2008/002824
- WO-A2-2008/116195
- JP-A- 2002 220 345
- JP-T- 2004 502 640
- JP-T- 2005 531 588
- JOMEI T.: 'Japanese Journal of Clinical Medicine', vol. 53, 22 June 1995, 1995 NEN, TOKUBETSUGO HIMANSHO, KABUSHIKI KAISHA, NIPPON RINSHOSHA, pages 354 - 358
- MATSUZAWA Y.: 'Igaku no Ayumi, Himansho - Metabolic Syndrome -Saishin Shinryo Consensus', 30 November 2005, ISHIYAKU PUB., INC., pages 11 - 16, XP008142154

## Description

### TECHNICAL FIELD

The present invention relates to a pharmaceutical composition for treating fatty liver disease, and more particularly relates to a pharmaceutical composition comprising a specific phenylglucitol derivative or a pharmaceutically acceptable salt thereof.

### BACKGROUND ART

Fatty liver disease, which is also called fatty liver, refers to a disease leading to liver injury caused by abnormal accumulation of fats (e.g., triglycerides) in liver cells. It is known that the early-stage pathology of fatty liver disease is simple fatty liver, which shows only fat deposition in liver cells, followed by development of steatohepatitis (including hepatic fibrosis) and further cirrhosis and/or hepatocellular carcinoma at more advanced stages. In general, possible causes of fat deposition in the liver include alcohol ingestion, obesity, diabetes, abnormal lipid metabolism, drugs (e.g., steroid, tetracycline), Cushing syndrome, poisoning (e.g., with white phosphorus), serious nutritional disorder, etc. The causes of fatty liver disease are classified into two major types, i.e., alcoholic and nonalcoholic, and the liver disease caused by the former is referred to as alcoholic liver disease (also called alcoholic liver injury), while the liver disease caused by the latter is referred to as nonalcoholic fatty liver disease (NAFLD). Alcoholic liver disease progresses from simple fatty liver at the early stage to steatohepatitis and/or cirrhosis at more advanced stages. Nonalcoholic fatty liver disease has been considered to remain at the stage of simple fatty liver without progressing to more advanced stages. In recent years, however, it has been shown that the pathology of nonalcoholic fatty liver disease may also progress from simple fatty liver to steatohepatitis and/or cirrhosis. Nonalcoholic fatty liver disease is defined as a disease with fat deposition in the liver, which occurs in patients whose alcohol ingestion history is not long enough to cause liver injury, except for cases of known etiology, such as viral hepatitis and autoimmune hepatitis. Nonalcoholic fatty liver disease is further classified into simple fatty liver, steatohepatitis and cirrhosis. Nonalcoholic steatohepatitis (NASH) refers to a pathology associated with inflammation, liver cell necrosis, ballooning and fibrosis, similarly to the case of alcoholic steatohepatitis (ASH). The onset of nonalcoholic simple fatty liver is induced by fat deposition in liver cells, and this fat accumulation is defined by the balance between increasing factors (influx and synthesis of fats in liver cells) and decreasing factors (catabolism of fats and their release from liver cells). Once damage of liver cells occurs, in addition to this fat deposition, nonalcoholic simple fatty liver will progress to nonalcoholic steatohepatitis. Nonalcoholic steatohepatitis is progressive and may finally progress to cirrhosis and hepatocellular carcinoma. Thus, nonalcoholic steatohepatitis is regarded as a serious type of nonalcoholic fatty liver disease. As described above, fatty liver disease is separated into alcoholic liver disease and nonalcoholic fatty liver disease, but these diseases have very similar histopathological features, for example, in each of the condition of simple fatty liver, steatohepatitis or cirrhosis. Thus, there is expected a common pathological mechanism to these diseases.

In the treatment of fatty liver disease, it is important to take away the causes and to improve fat accumulation in the liver. For the treatment of alcoholic liver disease, abstinence from alcohol is imperative, but it is difficult to achieve. On the other hand, most cases of nonalcoholic fatty liver disease are associated with insulin resistance, obesity, diabetes and hyperlipidemia, as expected from a possible onset mechanism for nonalcoholic fatty liver disease. If patients have these complications, they are first required to receive therapy for these complications. The therapeutic principle for nonalcoholic fatty liver disease is to improve lifestyle habits, including diet therapy and exercise therapy, which are however difficult to achieve securely under the present circumstances. In the case of nonalcoholic steatohepatitis, a more aggressive drug therapy is required because it is highly likely to progress to cirrhosis and/or hepatocellular carcinoma. Although some therapies have been attempted to improve oxidative stress and/or insulin resistance, which appear to be important for the onset and progress of nonalcoholic steatohepatitis, there is no therapy based on well-established scientific grounds under the present circumstances. In Japan, polyenylphosphatidylcholine (EPL) is used as a drug for simple fatty liver under medical insurance, but its therapeutic effect on nonalcoholic steatohepatitis has not yet been clarified. In view of the foregoing, no sufficient therapy has been established for fatty liver disease under the present circumstances, and there is a demand for the development of a highly effective therapeutic agent for fatty liver disease.

Under these circumstances, a document has been published, which discloses an inhibitor against the progress of diseases caused by abnormal fat accumulation in the liver, which comprises a sodium/glucose cotransporter (hereinafter referred to as SGLT) 2 inhibitor as an active ingredient (Patent Document 1). In this document, many O-glycoside compounds are listed as SGLT2 inhibitors, but there is no disclosure about compounds of formula (I) or pharmaceutically acceptable salts thereof. Moreover, there is no actual data showing their efficacy on the treatment of nonalcoholic steatohepatitis.

Likewise, another document has been published, which discloses combination therapy with an SGLT inhibitor and a PPAR agonist (Patent Document 2). This document discloses that T-1095, which is known as an SGLT inhibitor, reduced blood triglyceride levels in db/db mice. However, there is no disclosure about efficacy on the treatment of fatty liver disease.

Among compounds of formula (I), a compound in which R³ is azulen-2-yl, i.e., (1S)-1,5-anhydro-1-[5-(azulen-2-ylmethyl)-2-hydroxyphenyl]-D-glucitol shows SGLT inhibitory activity and hypoglycemic effect, and is disclosed to be useful as a therapeutic agent for various diabetes-related diseases (Patent Document 3). It is also disclosed that its choline salt has preferred properties as a pharmaceutical drug substance (Patent Document 4). However, there is no disclosure about efficacy on the treatment of fatty liver disease.

Among compounds of formula (I), a compound in which R³ is 1-benzothiophen-2-yl, i.e., (1S)-1,5-anhydro-1-[3-(1-benzothiophen-2-ylmethyl)-4-fluorophenyl]-D-glucitol shows SGLT inhibitory activity and hypoglycemic effect, and is disclosed to be useful as a therapeutic agent for various diabetes-related diseases (Patent Documents 5 and 6). It is also disclosed that its free form and its co-crystal with L-proline (at 1:1 molar ratio) have preferred properties as pharmaceutical drug substances (Patent Document 7). However, there is no disclosure about efficacy on the treatment of fatty liver disease.

Among compounds of formula (I), a compound in which R³ is 4-ethoxyphenyl, i.e., (1S)-1,5-anhydro-1-[4-chloro-3-(4-ethoxybenzyl)phenyl]-D-glucitol is disclosed to be usable as an SGLT inhibitor for treatment of obesity and type 2 diabetes (Patent Document 8). Moreover, its co-crystal with proline and its propylene glycol hydrate are also reported (Patent Document 9). However, there is no disclosure about efficacy on the treatment of fatty liver disease.
Patent Document 1: International Publication No. WO06/009149
Patent Document 2: International Publication No. WO02/080936
Patent Document 3: International Publication No. WO04/013118
Patent Document 4: International Publication No. WO07/007628
Patent Document 5: International Publication No. WO04/080990
Patent Document 6: International Publication No. WO05/012326
Patent Document 7: International Publication No. WO07/114475
Patent Document 8: International Publication No. WO03/099836
Patent Document 9: International Publication No. WO08/002824

### DISCLOSURE OF THE INVENTION

The present invention provides, (1S)-1,5-anhydro-1-[3-(1-benzothiophen-2-ylmethyl)-4-fluorophenyl]-D-glucitol or a pharmaceutically acceptable salt thereof, for use in a method for treating fatty liver disease, such as nonalcoholic fatty liver disease, nonalcoholic simple fatty liver or nonalcoholic steatohepatitis.

As a result of extensive and intensive studies on drugs for ameliorating fatty liver disease, the inventors of the present invention have found that (1S)-1,5-anhydro-1-[3-(1-benzothiophen-2-ylmethyl)-4-fluorophenyl]-D-glucitol or a pharmaceutically acceptable salt thereof, has an improving effect on abnormal accumulation of triglycerides in the liver and exerts an excellent therapeutic effect on fatty liver disease. This finding led to the completion of the present invention.

Namely, the present invention provides the following.
[1] The compound (1S)-1,5-anhydro-1-[3-1-benzothiophen-2-ylmethyl)-4-fluorophenyl]-D-glucitol or a phamnaceutically acceptable salt thereof for use in a method for treating fatty liver disease.
[2] The compound for use according to [1], wherein the (1S)-1,5-anhydro-1-[3-(1-benzothiophen-2-ylmethyl)-4-fluorophenyl]-D-glucitol or pharmaceutically acceptable salt thereof is (1S)-1,5-anhydro-1-[3-(1-benzothiophen-2-ylmethyl)-4-fluorophenyl]-D-glucitol.
[3] The compound for use according to [2], wherein the (1S)-1,5-anhydro-1-[3-(1-benzothiophen-2-ylmethyl)-4-fluorophenyl]-D-glucitol is a 1:1 molar ratio co-crystal of (1S)-1,5-anhydro-1-[3-(1-benzothiophen-2-ylmethyl)-4-fluorophenyl]-D-glucitol and L-proline.
[4] The compound for use according to any one of [1] to [3], wherein the fatty liver disease is nonalcoholic fatty liver disease, nonalcoholic simple fatty liver, or nonalcoholic steatohepatitis.

### BRIEF DESCRIPTION OF THE DRAWINGS

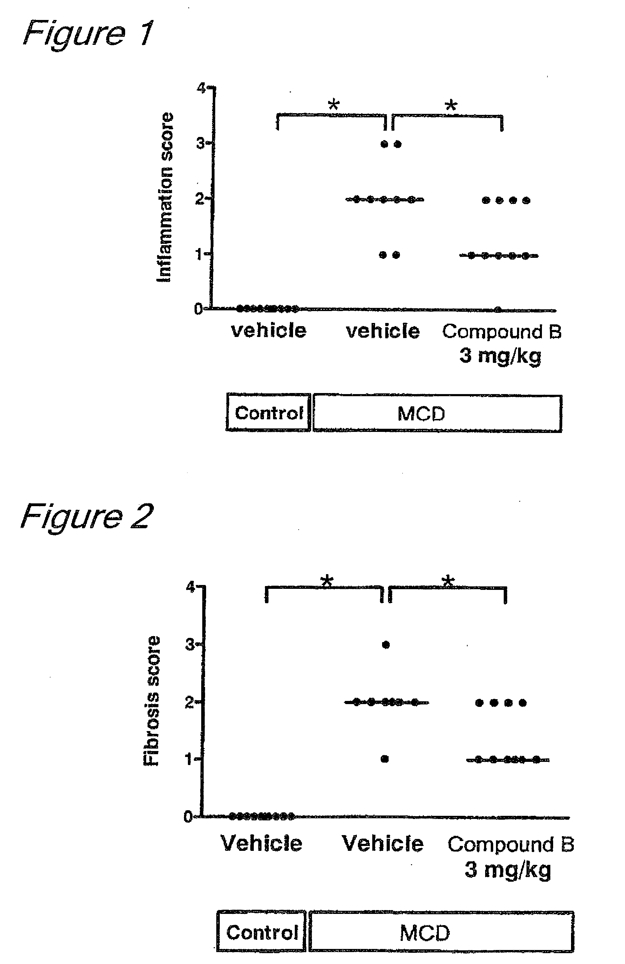

Figure 1 shows the results evaluated for the pathology of inflammatory cell infiltration (in MCD diet-fed rats). The median value for each evaluated group is indicated by a horizontal line (-) in the figure. It should be noted that an asterisk (*) in the figure indicates statistical significance over the second group.
Figure 2 shows the results evaluated for the pathology of hepatic fibrosis (in MCD diet-fed rats). The median value for each evaluated group is indicated by a horizontal line (-) in the figure. It should be noted that an asterisk (*) in the figure indicates statistical significance over the second group.

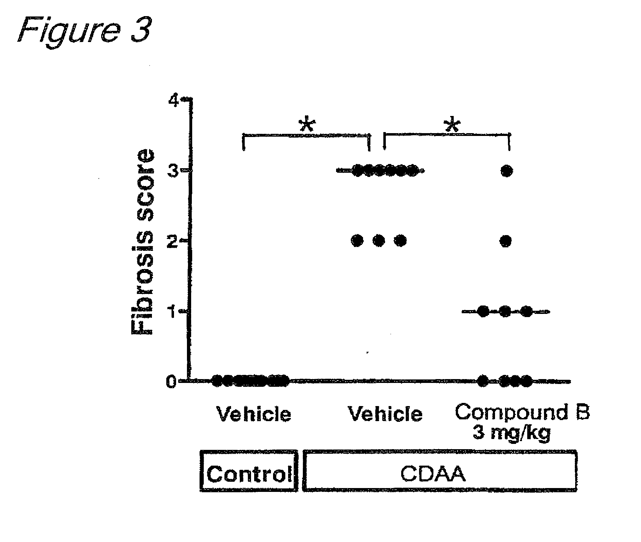

Figure 3 shows the results evaluated for the pathology of hepatic fibrosis (in CDAA diet-fed rats). The median value for each evaluated group is indicated by a horizontal line (-) in the figure. It should be noted that an asterisk (*) in the figure indicates statistical significance over the second group.

### BEST MODE FOR CARRYING OUT THE INVENTION

The present invention will be described in more detail below.

As used herein, the term "fatty liver disease," which is also called fatty liver, is intended to mean a disease leading to liver injury caused by abnormal fat accumulation in liver cells, as described in the BACKGROUND ART section. Moreover, fatty liver disease can be classified into alcoholic liver disease and nonalcoholic fatty liver disease. Diseases falling within the scope of fatty liver disease in the context of the present invention are summarized below.

Diseases falling within the scope of fatty liver disease include:
(1) Alcoholic liver disease (also called alcoholic liver injury): a disease caused by fat accumulation in liver cells as a result of alcohol ingestion. Examples include diseases such as alcoholic simple fatty liver, alcoholic steatohepatitis (ASH), alcoholic hepatic fibrosis, alcoholic cirrhosis and so on. It should be noted that alcoholic steatohepatitis is also called alcoholic fatty hepatitis and includes alcoholic hepatic fibrosis.
(2) Nonalcoholic fatty liver disease: a disease with fat deposition in the liver, which occurs in patients whose alcohol ingestion is not enough to cause liver injury, except for cases of known etiology, such as viral hepatitis and autoimmune hepatitis. Examples include diseases such as nonalcoholic simple fatty liver, nonalcoholic steatohepatitis (NASH), nonalcoholic hepatic fibrosis, nonalcoholic cirrhosis and so on.
(2-1) Nonalcoholic simple fatty liver: a disease only with fat deposition in liver cells.
(2-2) Nonalcoholic steatohepatitis (NASH): a disease with liver fatty change, along with inflammation, liver cell necrosis, ballooning and fibrosis, similarly to the case of alcoholic steatohepatitis, and also including nonalcoholic hepatic fibrosis.
(2-2-1) Nonalcoholic hepatic fibrosis: a disease with advanced fibrosis in liver tissues, along with excessive production and accumulation of collagen and other extracellular matrix components.
(2-3) Nonalcoholic cirrhosis: a disease with reconstructed hepatic lobule structure as a result of advanced fibrosis.

(1S)-1,5-anhydro-1-[3-(1-benzothiophen-2-ylmethyl)-4-fluorophenyl]-D-glucitol (hereinafter also referred to as Compound B) or a pharmaceutically acceptable salt thereof can be easily obtained, for example, as described in Patent Document 5 (supra) or in a manner obvious to those skilled in the art or according to modified methods thereof.

The structural formulae of Compounds A, B and C are shown below.

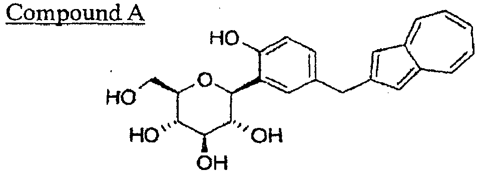

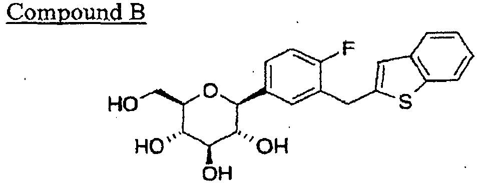

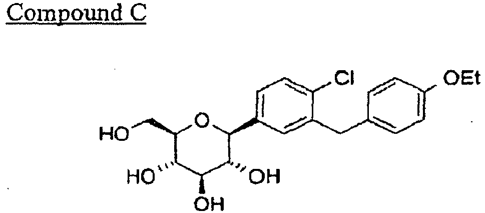

### (wherein Et represents an ethyl group)

As used herein, the term "pharmaceutically acceptable salt" is intended to mean an acid addition salt or a salt with a base, for example as described in Patent Document 3 or 5 (supra). Specific examples include acid addition salts with mineral acids (e.g., hydrochloric acid, hydrobromic acid, hydroiodic acid, sulfuric acid, nitric acid, phosphoric acid), organic acids (e.g., formic acid, acetic acid, propionic acid, oxalic acid, malonic acid, succinic acid, fumaric acid, maleic acid, lactic acid, malic acid, tartaric acid, citric acid, methanesulfonic acid, ethanesulfonic acid) or acidic amino acids (e.g., aspartic acid, glutamic acid); salts with inorganic bases (e.g., sodium, potassium, magnesium, calcium, aluminum), organic bases (e.g., methylamine, ethylamine, ethanolamine) or basic amino acids (e.g., lysine, ornithine); as well as ammonium salt, etc.

Moreover, the compound maybe present in any form, i.e., various hydrates, solvates, crystalline polymorphic substances or co-crystals. With respect to "(1S)-1,5-anhydro-1-[3-(1-benzothiophen-2-ylmethyl)-4-nuorophenyl]-D-glucitol or a pharmaceutically acceptable salt thereof," a preferred embodiment is a co-crystal of (1S)-1,5-anhydro-1-[3-(1-benzothiophen-2-ylmethyl)-4-fluorophenyl]-D-glucitol and L-proline (at 1:1 molar ratio), as described in Patent Document 7 (supra).

Furthermore, pharmaceutically acceptable prodrugs of the compound are also disclosed. The term "pharmaceutically acceptable prodrug" refers to a compound having a group which can be converted into a hydroxyl group or the like by solvolysis or under physiological conditions. Examples of a prodrug-forming group include those described in Prog. Med., 5, 2157-2161 (1985) or those described in "Development of Pharmaceuticals" (Hirokawa Publishing, 1990) vol. 7, Molecular Design 163-198.

A pharmaceutical preparation can be prepared in a conventional manner by using the compound or a pharmaceutically acceptable salt thereof and a pharmaceutical carrier, a pharmaceutical excipient or other additives commonly used for formulation purposes. Any mode of administration may be used, either oral administration in the dosage form of tablets, pills, capsules, granules, powders, solutions or the like, or parenteral administration in the dosage form of injections (e.g., intravenous or intramuscular injections) or suppositories or by the transnasal, transmucosal, percutaneous or other routes.

Solid compositions used for oral administration according to the present invention include tablets, powders, granules, etc. In these solid compositions, the compound or a pharmaceutically acceptable salt thereof is mixed with at least one inert diluent, for example, lactose, mannitol, glucose, hydroxypropylcellulose, microcrystalline cellulose, starch, polyvinylpyrrolidone, magnesium aluminometasilicate or the like. The compositions may also comprise additives in addition to the inert diluent(s), as exemplified by lubricants (e.g., magnesium stearate), disintegrants (e.g., calcium carboxymethyl cellulose), stabilizers, solubilizers and so on, as in the usual cases. Tablets or pills may optionally be coated with sugar coating or a gastric or enteric film, as exemplified by sucrose, gelatin, hydroxypropyl cellulose, hydroxypropyl methylcellulose phthalate or the like.

Liquid compositions for oral administration include pharmaceutically acceptable emulsions, solutions, suspensions, syrups, elixirs, etc., and comprise commonly-used inert diluents such as purified water or ethanol. These compositions may comprise, in addition to the inert diluents, auxiliaries (e.g., wetting agents, suspending agents), sweeteners, flavors, aromatics, and/or antiseptics.

Injections for parenteral administration comprise sterile aqueous or non-aqueous solutions, suspensions or emulsions. Examples of aqueous solutions or suspensions include injectable distilled water and physiological saline. Examples of non-aqueous solutions or suspensions include propylene glycol, polyethylene glycol, vegetable oils (e.g., olive oil), alcohols (e.g., EtOH), Polysorbate 80, etc. These compositions may further comprise auxiliaries such as antiseptics, wetting agents, emulsifiers, dispersants, stabilizers and/or solubilizers. They are sterilized, for example, by filtration through a bacteria-retaining filter, by incorporation with disinfectants or by irradiation. Alternatively, they may be formulated into sterile solid compositions and reconstituted for use by being dissolved in sterile water or a sterile injectable solvent before use.

Formulations for external use include ointments, plasters, creams, jellies, cataplasms, sprays, lotions, eye drops, eye ointments, etc. They comprise commonly-used ointment bases, lotion bases, aqueous or non-aqueous solutions, suspensions, emulsions or the like. Examples of ointment or lotion bases include polyethylene glycol, propylene glycol, white petrolatum, white beeswax, polyoxyethylene hydrogenated castor oil, glycerine monostearate, stearyl alcohol, cetyl alcohol, Lauromacrogol, sorbitan sesquioleate and so on.

Transmucosal formulations such as inhalants or transnasal formulations are used in solid, liquid or semi-solid form and can be prepared in a conventionally known manner. For example, such formulations may be supplemented as appropriate with known excipients and further with pH adjustors, antiseptics, surfactants, lubricants, stabilizers, thickeners and so on. For their administration, an appropriate device for inhalation or insufflation may be used. For example, using a known device (e.g., a metered-dose inhalation device) or a nebulizer, each compound may be administered alone or as a powder of a formulated mixture or as a solution or suspension in combination with a pharmaceutically acceptable carrier. Dry powder inhalators or the like may be for single or multiple administration use, and dry powders or powder-containing capsules may be used in such devices. Alternatively, they may be in the form of pressurized aerosol sprays which use an appropriate propellant, for example, a preferred gas such as chlorofluoroalkane, hydrofluoroalkane or carbon dioxide.

In general, for oral administration, the daily dosage is desirably about 0.001 to 100 mg/kg, preferably 0.1 to 30 mg/kg, and more preferably 0.1 to 10 mg/kg body weight, given as a single dose or in 2 to 4 divided doses. For intravenous administration, the daily dosage is desirably about 0.0001 to 10 mg/kg body weight, given in one or several doses per day. Likewise, for transmucosal formulations, the daily dosage is about 0.001 to 100 mg/kg body weight, given in one or several doses per day. The dosage may be determined as appropriate for each case in consideration of symptom, age, sex and so on.

It should be noted that a pharmaceutical preparation based on the compound of the present invention can be used in combination with other drugs which are used for treatment of fatty liver disease. For example, drugs which can be used in combination with this pharmaceutical preparation include biguanides (e.g., metformin), thiazolidine derivatives (e.g., pioglitazone hydrochloride), α-glucosidase inhibitors (e.g., voglibose), insulin secretagogues (e.g., nateglinide), vitamins, eicosapentaenoic acid (EPA), betaine, N-acetylcysteine (NAC), fibrate drugs (e.g., bezafibrate), HMG-CoA reductase inhibitors (e.g., atorvastatin), probucol, ursodeoxycholic acid (UDCA), taurine, stronger neo-minophagen C, polyenephosphatidylcholine, angiotensin II receptor antagonists (e.g., losartan) or bofutsushosan (oriental herbal medicine), etc. In such combination use, drugs may be administered simultaneously or separately in succession or at desired time intervals. Formulations for simultaneous administration may be in either mixed or separate form.

### EXAMPLES

### Example 1÷ (reference):

### Effect on nonalcoholic simple fatty liver model (KK-A^{y} mice) (1)

### <Test method>

KK-A^{y} mice (female, purchased from CLEA Japan, Inc.) were used. The mice were fed with CMF (for special breeding, purchased from Oriental Yeast Co., Ltd., Japan) ad libitum. At 14 weeks of age, they were measured for their body weight, blood glucose levels, plasma insulin levels, plasma triglyceride levels and plasma alanine aminotransferase (ALT), and then divided into two groups such that these items were equal between the groups (8 animals per group). The first group was administered with vehicle (0.5% methylcellulose) at a dose of 10 mL/kg, and the second group was administered with a choline salt of (1S)-1,5-anhydro-1-[5-(azulen-2-ylmethyl)-2-hydroxyphenyl]-D-glucitol (i.e., a choline salt of Compound A) at a dose of 3 mg/kg (calculated as Compound A), each being administered orally once a day for 2 weeks. On the day following the final administration, the liver was collected from each mouse under ether anesthesia, frozen in liquid nitrogen and then stored at -80°C.

Liver triglyceride content was measured in the following manner.
1. A portion (50 to 150 mg) of each liver frozen and stored at -80°C was taken to an Assist tube.
2. After addition of methanol (2 mL), the liver sample was homogenized with a POLYTRON (KINEMATICA).
3. To the homogenate, chloroform (4 mL) was added and vigorously stirred at room temperature for 10 minutes.
4. Milli-Q water (1 mL) was further added and vigorously stirred.
5. The sample was centrifuged in a low speed centrifuge (Hitachi, Ltd., Japan) (2,500 rpm, 5 min, room temperature).
6. A portion of the lower layer (total volume: 4.5 mL) was taken to an Eppendorf tube and evaporated in a centrifugal evaporator (Sakuma Seisakusho, Japan) to remove the solvent.
7. The residue in the Eppendorf tube was dissolved again by addition of ethanol (10 µL).
8. Triglyceride E-Test Wako reagent (1 mL, Wako Pure Chemical Industries, Ltd., Japan) was added to the resulting solution, and triglycerides were quantified.
9. From the result obtained above, the triglyceride content per g of liver was calculated for each sample. The data were expressed as mean ± standard error.

### <Results>

The results obtained are as shown in Table 1. In comparison with normal mice whose liver triglyceride content is 5 to 10 (mg/g liver), KK-A^{y} mice have a higher liver triglyceride content and can be diagnosed as having fatty liver. Upon administration of Compound A, the liver triglyceride content in KK-A^{y} mice was significantly improved.

This result indicates that Compound A is useful as a therapeutic agent for nonalcoholic simple fatty liver.

**[Table 1]**

| Group | Test drug | Liver triglyceride content (mg/g liver) |
|---|---|---|
| First group | Vehicle | 60.0 ± 8.4 |
| Second group | Compound A 3 mg/kg | 30.7 ± 5.4* |

| | | |
|---|---|---|
| * indicates statistical significance over the first group. | | |

### Example 2:

### Effect on nonalcoholic simple fatty liver model (KK-A^{y} mice) (2)

### <Test method>

The test was conducted in the same manner as shown in Example 1, except that this test was conducted with 3 groups of 8 animals, and the first group was administered with vehicle (0.5% methylcellulose) at a dose of 10 mL/kg, the second group was administered with a co-crystal (1:1 molar ratio) of (1S)-1,5-anhydro-1-[3-(1-benzothiophen-2-ylmethyl)-4-fluorophenyl]-D-glucitol (Compound B) and L-proline at a dose of 3 mg/kg (calculated as Compound B), and the third group was administered with a control compound, 2-(4-methoxybenzyl)phenyl 6-O-ethoxycarbonyl-β-D-glucopyranoside (hereinafter also referred to as Compound X, whose structural formula is shown below) disclosed in Patent Document 1 (supra) at a dose of 36 mg/kg, each being administered orally once a day for 2 weeks. Then, the liver triglyceride content was measured for each group in the same manner as shown in Example 1.

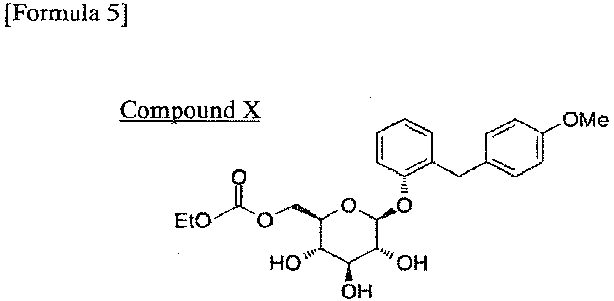

### (wherein Me represents a methyl group, and Et represents an ethyl group)

### <Results>

The results obtained are as shown in Table 2. Although KK-A^{y} mice had fatty liver, as in the case of Example 1, their liver triglyceride content was significantly improved upon administration of Compound B. This result indicates that Compound B is useful as a therapeutic agent for nonalcoholic simple fatty liver.

In contrast, Compound X showed no significant effect in spite of being administered at a dose 10-fold or higher than that of Compound B.

**[Table 2]**

| Group | Test drug | Liver triglyceride content (mg/g liver) |
|---|---|---|
| First group | Vehicle | 52.8 ± 4.9 |
| Second group | Compound B 3 mg/kg | 34.8 ± 1.9* |
| Third group | Compound X 36 mg/kg | 57.9 ± 4.5 |

| | | |
|---|---|---|
| * indicates statistical significance over the first group. | | |

### Example 3:

### Effect on nonalcoholic simple fatty liver model (KK-A^{y} mice) (3)

### <Test method>

The test was conducted in the same manner as shown in Example 1, except that this test was conducted with 3 groups of 8 animals, and the first group was administered with vehicle (0.5% methylcellulose) at a dose of 10 mL/kg, the second group was administered with a co-crystal (1:1 molar ratio) of Compound B and L-proline at a dose of 3 mg/kg (calculated as Compound B), and the third group was administered with a control compound, T-1095 disclosed in Patent Document 2 (supra), i.e., 3-(benzo[b]furan-5-yl)-2',6'-dihydroxy-4'-methylpropiophenone 2'-O-(6-O-methoxycarhonyl)-β-D-glucopyranoside (hereinafter also referred to as Compound Y, whose structural formula is shown below) at a dose of 34 mg/kg, each being administered orally once a day for 2 weeks. Then, the liver triglyceride content was measured for each group in the same manner as shown in Example 1.

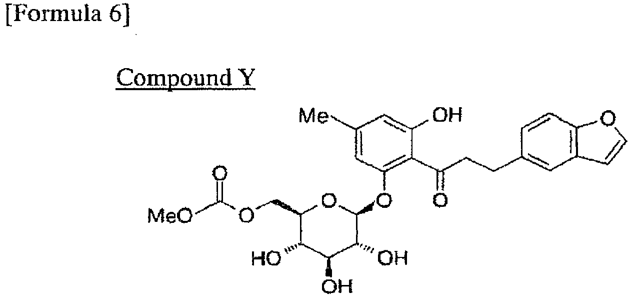

### (wherein Me represents a methyl group)

### <Results>

The results obtained are as shown in Table 3. Although KK-A^{y} mice had fatty liver, as in the case of Example 1, their liver triglyceride content was significantly improved upon administration of Compound B.

In contrast, Compound 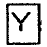 showed no significant effect in spite of being administered at a dose 10-fold or higher than that of Compound B.

**[Table 3]**

| Group | Test drug | Liver triglyceride content (mg/g liver) |
|---|---|---|
| First group | Vehicle | 59.7 ± 6.2 |
| Second group | Compound B 3 mg/kg | 41.2 ± 5.1* |
| Third group | Compound Y 34 mg/kg | 59.7 ± 8.9 |

| | | |
|---|---|---|
| * indicates statistical significance over the first group. | | |

### Example 4:

### Effect on nonalcoholic steatohepatitis model (methionine/choline-deficient diet (MCD diet)-fed rats) (1)

### <Test method>

This test was conducted by reference to a document (J Hepatol., 2003, 39, 756-764). Wistar rats (male, purchased from Charles River Japan, Inc.) were used. The rats were fed with MCD diet (methionine/choline-deficient diet, MP Biochemicals) or normal control diet (methionine/choline control diet, MP Biochemicals) ad libitum. At 9 weeks of age, they were measured for their body weight and divided into groups of equal body weight, followed by initiation of the test (10 animals per group). The first group was fed with normal control diet and administered with vehicle (0.5% methylcellulose) at a dose of 5 mL/kg. The second and third groups were fed with MCD diet, and the second group was administered with vehicle (0.5% methylcellulose) at a dose of 5 mL/kg, while the third group was administered with a co-crystal (1:1 molar ratio) of Compound B and L-proline at a dose of 3 mg/kg (calculated as Compound B). Each drug was administered orally once a day for 16 weeks. On the day following the final administration, the liver was collected from each rat under ether anesthesia and a portion of the liver was fixed in 10% neutral buffered formalin. Paraffin sections (3 µm) were prepared in a standard manner and subjected to HE staining and van Gieson staining. The HE-stained specimens were used for evaluation of inflammatory lesions, while the van Gieson-stained specimens were used for evaluation of fibrosis. Evaluation was made by reference to the NASH activity score (NAS) for inflammatory lesions and to the Brunt classification for fibrosis (Clinical Practice Guidelines for NASH/NAFLD, edited by the Japan Society of Hepatology, 2006), based on a five-point scale of 0, 1, 2, 3 and 4 (see Table 4). In Table 4, it should be noted that a visual field at "200-fold magnification" corresponds to the 1/4 area of a visual field at "100-fold magnification."

**[Table 4]**

| Evaluation (Score) | Inflammatory lesion | Fibrosis |
|---|---|---|
| 0 | None | None |
| 1 | 1 lesion at 100-fold magnification | Limited to around central veins |
| 2 | 2 to 4 lesions at 100-fold magnification | Also found around Glisson's capsules |
| 3 | 2 to 4 lesions at 200-fold magnification | Associated with bridging fibrosis |
| 4 | 5 or more lesions at 200-fold magnification | Nodular transformation |

### <Results>

The results obtained are as shown in Figures 1 and 2. The rats fed with MCD diet showed significant increases in the pathological scores of inflammatory cell infiltration and hepatic fibrosis over the rats fed with normal control diet, thus indicating that they had the condition of nonalcoholic steatohepatitis. Upon administration of Compound B, the pathological scores of inflammatory cell infiltration and hepatic fibrosis in this model were significantly improved. This result indicates that Compound B is useful as a therapeutic agent for nonalcoholic steatohepatitis.

### Example 5:

### Effect on nonalcoholic steatohepatitis model (choline-deficient L-amino acid-defined diet (CDAA diet)-fed rats) (1)

### <Test method>

This test was conducted by reference to a document (Biochem Biophys Res Commun., 2004, 315(1), 187-195). Wistar rats (male, purchased from Charles River Japan, Inc.) were used. The rats were fed with CDAA diet (choline-deficient L-amino acid-defined diet (Choline Deficient and Iron Supplemented L-Amino Acid Defined Rat Diet, Dyets)) or normal control diet (Choline and Iron Supplemented L-Amino Acid Defined Rat Diet, Dyets) ad libitum. At 9 weeks of age, they were measured for their body weight and divided into groups of equal body weight, followed by initiation of the test (10 animals per group). The first group was fed with normal control diet and administered with vehicle (0.5% methylcellulose) at a dose of 5 mL/kg. The second and third groups were fed with CDAA diet, and the second group was administered with vehicle (0.5% methylcellulose) at a dose of 5 mL/kg, while the third group was administered with a co-crystal (1:1 molar ratio) of Compound B and L-proline at a dose of 3 mg/kg (calculated as Compound B). Each drug was administered orally once a day for 5 weeks. On the day following the final administration, the liver was collected from each rat under ether anesthesia. After a portion of the liver was fixed in 10% neutral buffered formalin, paraffin sections (3 µm) were prepared in a standard manner and subjected to van Gieson staining. Fibrosis was evaluated by reference to the Brunt classification (Clinical Practice Guidelines for NASH/NAFLD, edited by the Japan Society of Hepatology, 2006), based on a five-point scale of 0, 1, 2, 3 and 4 (see Table 4).

### <Results>

The results obtained are as shown in Figure 3. The rats fed with CDAA diet showed a significant increase in the pathological score of hepatic fibrosis over the rats fed with normal control diet, thus indicating that they had the condition of nonalcoholic steatohepatitis. Upon administration of Compound B, the pathological score of hepatic fibrosis in this model was significantly improved. This result indicates that Compound B is useful as a therapeutic agent for nonalcoholic steatohepatitis.

The above results indicated that upon administration of the compound or pharmaceutically acceptable salts thereof, abnormal accumulation of triglycerides in the liver (simple fatty liver) was improved and further the condition of nonalcoholic steatohepatitis (inflammation and fibrosis) caused by abnormal accumulation of triglycerides in the liver was also improved. In general, alcoholic liver disease and nonalcoholic fatty liver disease have very similar histopathological features in each condition of simple steatosis, steatohepatitis (including hepatic fibrosis) and cirrhosis in fatty liver disease, and are expected to have a common pathological mechanism. Thus, it is evident that the pharmaceutical compositions of the present invention are useful as therapeutic agents for fatty liver disease. Moreover, the compounds of formula (I) or pharmaceutically acceptable salts thereof were confirmed to have a higher improving effect on abnormal accumulation of triglycerides in the liver than the compounds (Compound X and Compound Y) disclosed in Patent Documents 1 and 2 (supra). This result suggests that the compounds of formula (I) or pharmaceutically acceptable salts thereof can also be expected to have a higher effect on nonalcoholic steatohepatitis than the compounds (Compound X and Compound Y) disclosed in Patent Documents 1 and 2 (supra).

### INDUSTRIAL APPLICABILITY

(1S)-1,5-anhydro-1-[3-(1-benzothiophen-2-ylmethyl)-4-fluorophenyl]-D-glucitol or a pharmaceutically acceptable salt thereof, has an improving effect on abnormal accumulation of triglycerides in the liver and can be used as a pharmaceutical composition for treating fatty liver disease, such as nonalcoholic fatty liver disease in one embodiment, or nonalcoholic simple fatty liver and/or nonalcoholic steatohepatitis in another embodiment.

## Claims

1. The compound (1S)-1,5-anhydro-1-[3-(1-benzothiophen-2-ylmethyl)-4-fluorophenyl]-D-glucitol or a pharmaceutically acceptable salt thereof for use in a method for treating fatty liver disease.

2. The compound for use according to claim 1, wherein the (1S)-1,5-anhydro-1-[3-(1-benzothiophen-2-ylmethyl)-4-fluorophenyl]-D-glucitol or pharmaceutically acceptable salt thereof is (1S)-1,5-anhydro-l-[3-(1-benzothiophen-2-ylmethyl)-4-fluorophenyl]-D-glucitol.

3. The compound for use according to claim 2, wherein the (1S)-1,5-anhydro-1-[3-(1-benzothiophen-2-ylmethyl)-4-fluorophenyl]-D-glucitol is a 1:1 molar ratio co-crystal of (1S)-1,5-anhydro-1-[3-(1-benzothiophen-2-ylmethyl)-4-fluorophenyl]-D-glucitol and L-proline.

4. The compound for use according to any one of claims 1 to 3, wherein the fatty liver disease is nonalcoholic fatty liver disease, nonalcoholic simple fatty liver, or nonalcoholic steatohepatitis.

## Patentansprüche

1. Verbindung (1S)-1,5-Anhydro-1-[3-(1-benzothiophen-2-ylmethyl)-4-fluorphenyl]-D-glucitol oder ein pharmazeutisch annehmbares Salz davon zur Verwendung in einem Verfahren zur Behandlung einer Fettlebererkrankung.

2. Verbindung zur Verwendung gemäss Anspruch 1, wobei das (1S)-1,5-Anhydro-1-[3-(1-benzothiophen-2-ylmethyl)-4-fluorphenyl]-D-glucitol oder ein pharmazeutisch annehmbares Salz davon (1S)-1,5-Anhydro-1-[3-(1-benzothiophen-2-ylmethyl)-4-fluorphenyl]-D-glucitol ist.

3. Verbindung zur Verwendung gemäss Anspruch 2, wobei das (1S)-1,5-Anhydro-1-[3-(1-benzothiophen-2-ylmethyl)-4-fluorphenyl]-D-glucitol ein Cokristall von (1S)-1,5-Anhydro-1-[3-(1-benzothiophen-2-ylmethyl)-4-fluorphenyl]-D-glucitol und L-Prolin im molaren Verhältnis 1:1 ist.

4. Verbindung zur Verwendung gemäss irgendeinem der Ansprüche 1 bis 3, wobei die Fettlebererkrankung eine nicht-alkoholische Fettlebererkrankung, eine einfache nicht-alkoholische Fettlebererkrankung oder nicht-alkoholische Steatohepatitis ist.

## Revendications

1. Composé (1S)-1,5-anhydro-1-[3-(1-benzothiophén-2-ylméthyl)-4-fluorophényl]-D-glucitol ou un sel pharmaceutiquement acceptable de celui-ci pour une utilisation dans un procédé de traitement d'une stéatose hépatique.

2. Composé pour une utilisation selon la revendication 1, dans lequel le (1S)-1,5-anhydro-1-[3-(1-benzothiophén-2-ylméthyl)-4-fluorophényl]-D-glucitol ou le sel pharmaceutiquement acceptable de celui-ci est le (1S)-1,5-anhydro-1-[3-(1-benzothiophén-2-ylméthyl)-4-fluorophényl]-D-glucitol.

3. Composé pour une utilisation selon la revendication 2, dans lequel le (1S)-1,5-anhydro-1-[3-(1-benzothiophén-2-ylméthyl)-4-fluorophényl]-D-glucitol est un co-cristal de rapport molaire 1/1 de (1S)-1,5-anhydro-1-[3-(1-benzothiophén-2-ylméthyl)-4-fluorophényl]-D-glucitol et de L-proline.

4. Composé pour une utilisation selon l'une quelconque des revendications 1 à 3, dans lequel la stéatose hépatique est une stéatose hépatique non alcoolique, une stéatose hépatique simple non alcoolique ou une stéatohépatite non alcoolique.
